# EUROPEAN PATENT SPECIFICATION

(11) **EP 0 261 470 B1**
(45) Date of publication and mention of the grant of the patent: **05.05.1993**
(21) Application number: 87112860.9
(22) Date of filing: 03.09.1987
(51) Int. Cl.: A61L 17/00

(54) **Bioabsorbable coating for a surgical article**
Bioresorbierbare Beschichtung für chirurgischen Artikel
Revêtement bioabsorbable pour article chirurgical

(30) Priority: 23.09.1986 US 910598
(43) Date of publication of application: 30.03.1988
(73) Proprietor: AMERICAN CYANAMID COMPANY, Wayne, NJ 07470-8426 (US)
(72) Inventor: Jarrett, Peter Kendrick, Trumbull Connecticut 06611 (US); Casey, Donald James, Ridgefield Connecticut 06877 (US); Lehmann, Leonard Theodore, Danbury Connecticut 06811 (US)
(74) Representative: Wächtershäuser, Günter, Prof. Dr.

(56) References cited:
- EP-A- 0 214 861
- US-A- 3 867 190
- US-A- 3 942 532

## Description

The invention refers to a suture or ligature with a bioabsorbable coating of a copolymer comprising the monomer caprolactone.

Such a suture or ligature is known from US-A-3 867 190.

It is the problem of this invention to advance sutures or ligatures of this kind by improving the knot security and the knot repositioning characteristics. This problem is solved by the suture or ligature of Claim 1.

The sutures or ligatures of this invention are less stiff than sutures using a coating described in the prior art. Further, tissue drag appears to be equal to, if not better than, suture coatings disclosed in the prior art. Moreover, the coating of this invention does not present a hazy appearance on a suture.

In a special embodiment, the inherent viscosity of the random copolymer is 0.2 to 1.4 dl/g (0.5 g/dl in CHCl₃, 30°C)
The surgical article coated with the above described polymers can be bioabsorbable. In one embodiment, the invention is a coating in combination with a bioabsorbable suture or ligature. In a specific embodiment, the suture or ligature is manufactured from a polymer prepared from one or more monomers selected from the group consisting of lactide, carbonates and lactones.

In a more specific embodiment, the suture or ligature is manufactured from a homopolymer prepared from the monomer glycolide or from a copolymer prepared from the monomers glycolide and either or both 1,3-dioxan-2-one and lactide.

The coating in combination with the suture or ligature can be in multifilamentary form. In a specific embodiment, the coating comprises 1/10 to 5% by weight of the coated multifilamentary suture or ligature. In a more specific embodiment, the coating comprises 1/2 to 3% by weight of the coated multifilamentary suture or ligature. In the more specific embodiment, the coating can comprise up to 1 1/2 percent by weight of the coated multifilamentary suture or ligature.

A process for manufacturing a coating in combination with a bioabsorbable surgical article has also been invented in accordance with Claim 8.

### Description of the Preferred Embodiment(s)

The following examples describe the best mode of the invention. Unless otherwise specified, all of the inherent viscosity measurements in the examples were conducted at 30°C.

### COMPARATIVE EXAMPLE A

### ε-Caprolactone Homopolymer

A sample of ε-caprolactone homopolymer was purchased from Scientific Polymer Products, Inc. The sample ^{η}inh was measured as 0.27 dl/g (0.5 g/dl in CHCl₃). GPC analysis in CH₂Cl₂ using polystyrene standards gave MW = 17,600 and MN = 8500.

### COMPARATIVE EXAMPLE B

### Synthesis of ε-Caprolactone Homopolymer

ε-Caprolactone (10g, 0.088 mole), lauryl alcohol (0.122g, 6.57 x 10⁻⁴ mole) and stannous chloride dihydrate (0.988 mg, 4.38 x 10⁻⁶ mole) were combined in a flask. The flask was flushed with nitrogen and evacuated. The flask was heated at 135°c in an oil bath for 24 hours. The resulting polymer had an ^{η}inh of 0.53 dl/g (0.5 g/dl in HFAS). GPC analysis in CH₂Cl₂ using polystyrene standards gave MW = 65,200 and MN = 26,900.

### EXAMPLE 1

### Synthesis of ε-Caprolactone-1-Lactide Copolymer

ε-Caprolactone (212.5g, 1.86 mole), 1-lactide (37.5 g, 0.31 mole), lauryl alcohol (4.10 ml, 0.018 mole) and stannous chloride dihydrate (35.9 mg, 1.59 x 10⁻⁴ mole) were combined in a stirred reactor under nitrogen at 175°C. The mixture was stirred at 175°C for 3 hours. The resulting polymer had a composition, as determined by H'NMR, of 84 wt. % ε-caprolactone and 16 wt. % 1-lactide. The inherent viscosity of the copolymer was 0.50 dl/g (0.5 g/dl in CHCl₃).

### EXAMPLE 2

### Synthesis of ε-Caprolactone-Glycolide Copolymer

ε-Caprolactone (170 g, 1.49 mole), glycolide (30 g, 0.26 mole), lauryl alcohol (1.37 g, 7.3 x 10⁻³ mole) and stannous octoate (0.052 g, 1.2 x 10⁻⁴ mole) were combined in a stirred reactor under nitrogen at 180°C. The mixture was stirred at 180°C for 4.5 hours. The resulting polymer had an inherent viscosity of 0.68 dl/g (0.5 g/dl in CHCl₃). The composition was determined by H'NMR to be 85 wt. % ε-caprolactone and 15 wt. % glycolide.

### EXAMPLES 3-6

### Synthesis of ε-Caprolactone-Glycolide Copolymers

A series of ε-caprolactone-glycolide copolymers was prepared by the general procedure described in Example 2. Specific preparative details and properties of the resulting polymers are summarized in Table 1.

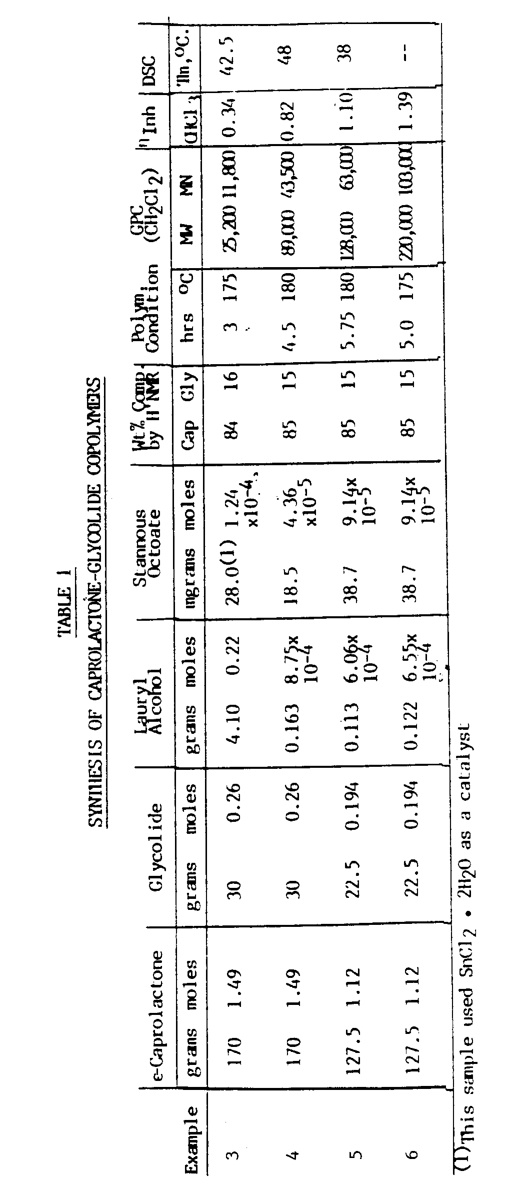

Table 2 summarizes the in vitro performance for the bioabsorbable coatings of this invention.

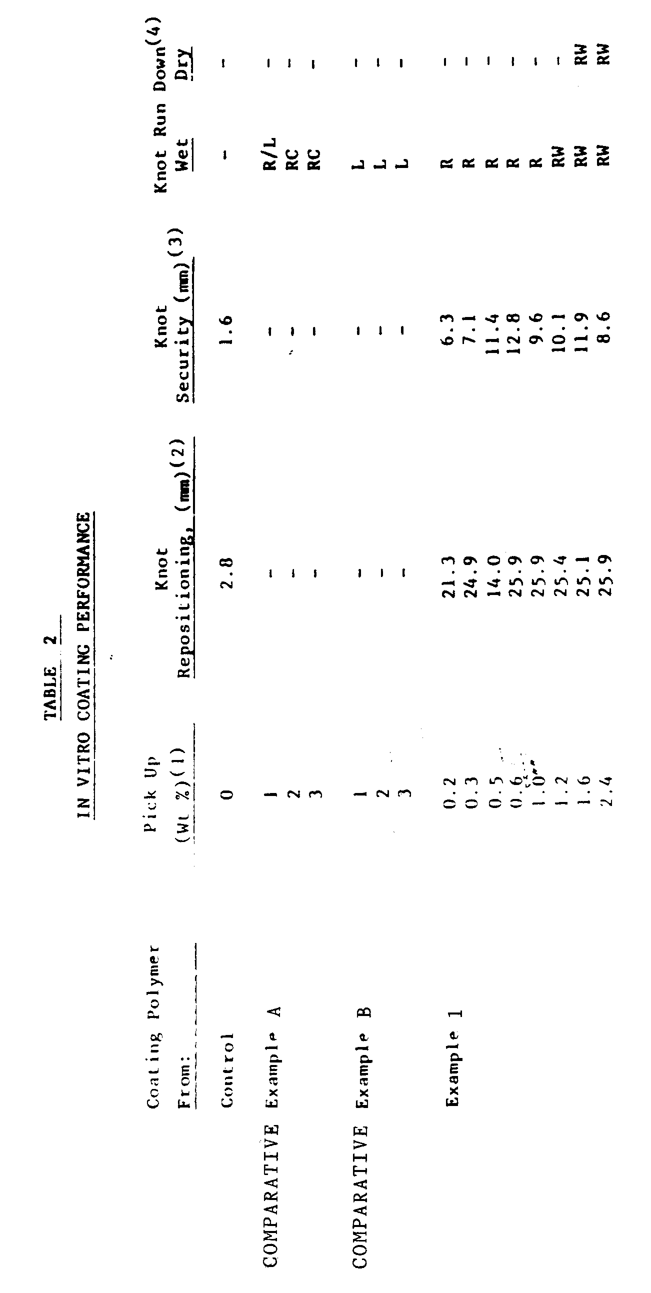

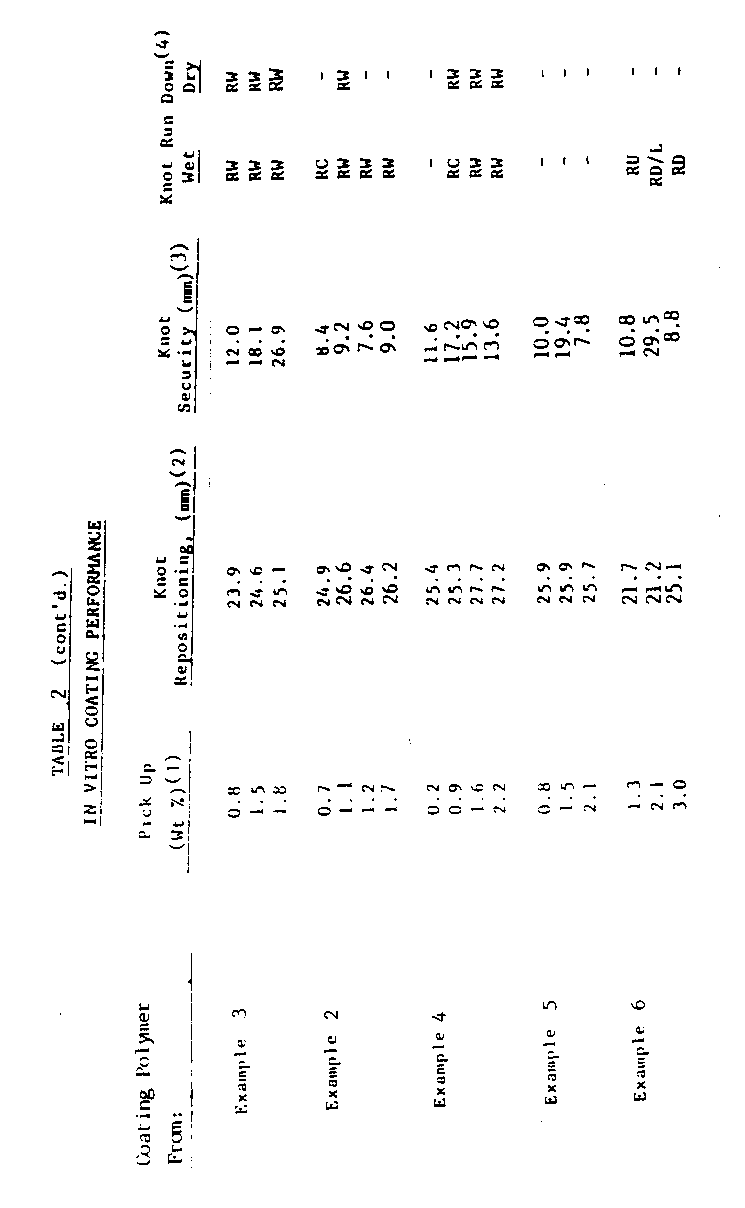

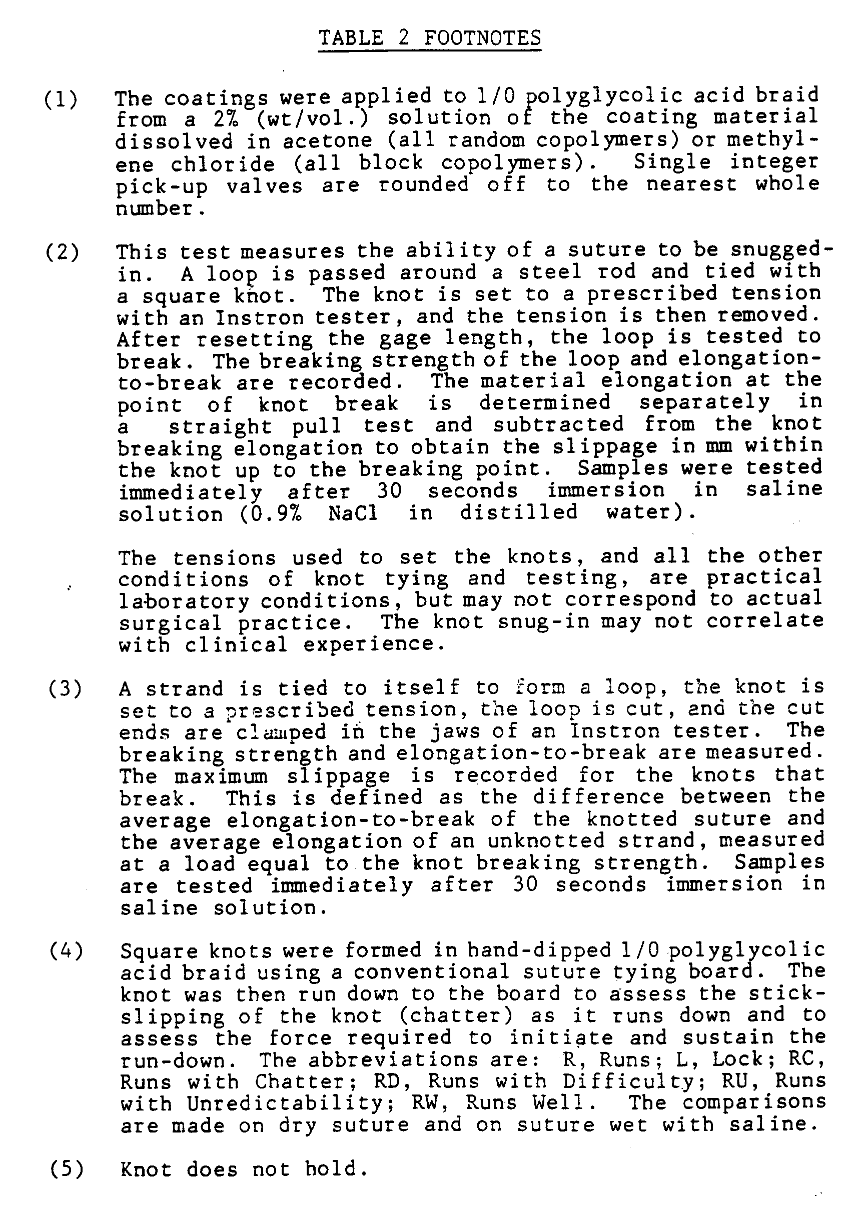

Table 3 summarizes the in vivo performance for some of the bioabsorbable coatings of this invention.

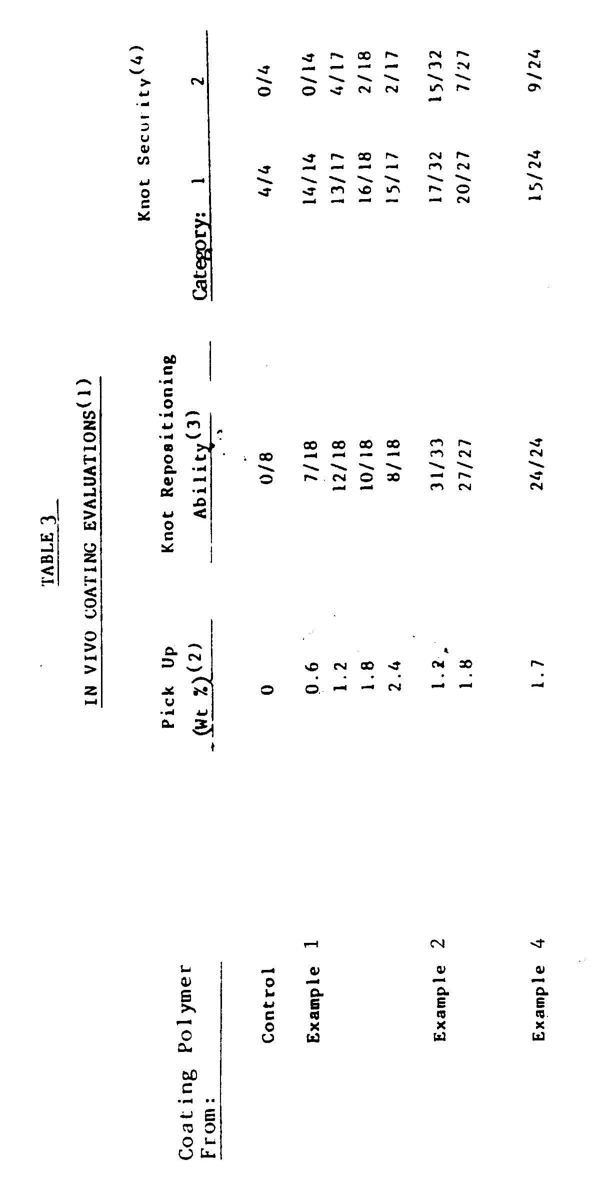

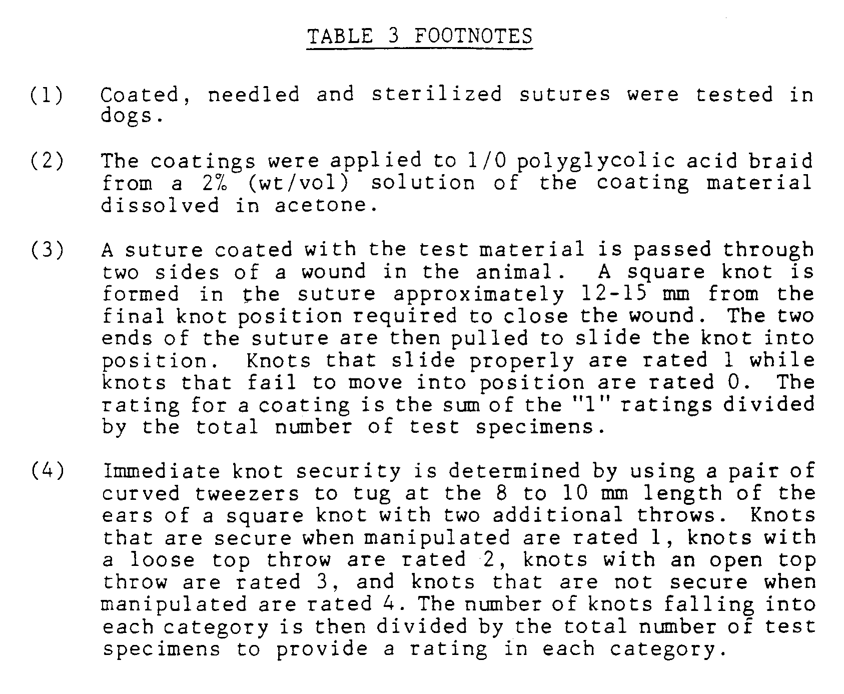

## Claims

1. A surgical suture or ligature with good knot security and knot repositioning characteristics having a bioabsorbable coating comprising a random copolymer with 70 to 85 wt-% of units of formula (I): 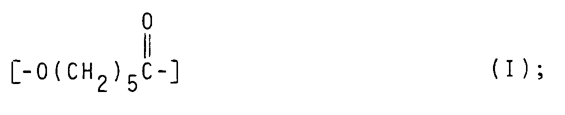 the remaining units having the formula (II) 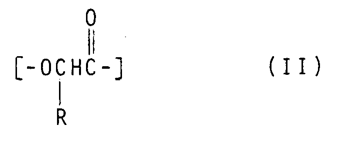 wherein R is H or CH₃.

2. A suture or ligature of Claim 1, wherein the remaining units comprise the formula (II) wherein R is H.

3. A suture or ligature of Claim 1, wherein the inherent viscosity of said copolymer is 0.2 to 1.4 dl/g (0.5 g/dl in CHCl₃, 30°C).

4. A suture or ligature of Claim 1 wherein the suture or ligature is manufactured from a bioabsorbable polymer prepared from one or more monomers selected from the group consisting of lactides, carbonates and lactones.

5. A suture or ligature of Claim 3 wherein the suture or ligature is manufactured from a homopolymer prepared from the monomer glycolide, or from a copolymer prepared from the monomers glycolide and either or both 1,3-dioxan-2-one and lactide.

6. A suture or ligature of Claim 4 wherein the suture or ligature is in multifilamentary form.

7. A suture or ligature of Claim 5 wherein the coating comprises about 1/10 to 5 % by weight of the coated suture or ligature.

8. A process for manufacturing a coated suture or ligature of one of Claims 1 to 7 comprising
dissolving in acetone the random copolymer;
contacting the suture or ligature with the dissolved copolymer;
maintaining the contact between said suture or ligature and dissolved copolymer until the copolymer on said suture or ligature comprises from 1/10 to 5 % by weight of the coated suture or ligature;
removing said coated suture or ligature from said dissolved copolymer; and
drying the copolymer on said suture or ligature.

## Patentansprüche

1. Ein chirurgisches Naht- oder Ligaturmaterial mit guter Knotensicherheit und guten Knoten-Repositionierungseigenschaften sowie einem bioresorbierbaren Überzug, umfassend ein statistisches Copolymer mit 70 bis 85 Gew.-% von Einheiten der Formel (I): 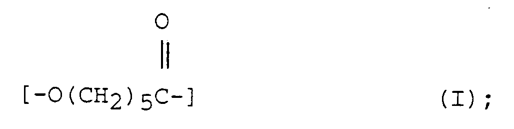 wobei die verbleibenden Einheiten die Formel (II) haben 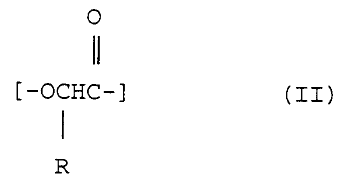 worin R H oder CH₃ ist.

2. Ein Naht- oder Ligaturmaterial nach Anspruch 1, worin die verbleibenden Einheiten die Formel (II) umfassen, worin R = H ist.

3. Ein Naht- oder Ligaturmaterial nach Anspruch 1, worin die Grenzviskositätszahl des genannten Copolymers 0,2 bis 1,4 dl/g (0,5 g/dl in CHCl₃, 30°C) beträgt.

4. Ein Naht- oder Ligaturmaterial nach Anspruch 1, worin das Naht- oder Ligaturmaterial hergestellt ist aus einem bioresorbierbaren Polymer, hergestellt aus einem oder mehreren Monomeren, ausgewählt aus der Gruppe bestehend aus Lactiden, Carbonaten und Lactonen.

5. Ein Naht- oder Ligaturmaterial nach Anspruch 3, worin das Naht- oder Ligaturmaterial hergestellt ist aus einem Homopolymer, zubereitet aus dem Monomer Glykolid oder aus einem Copolymer, zubereitet aus den Monomeren Glykolid und 1,3-Dioxan-2-on und/oder Lactid.

6. Ein Naht- oder Ligaturmaterial nach Anspruch 4, worin das Naht- oder Ligaturmaterial in Multifilamentform vorliegt.

7. Ein Naht- oder Ligaturmaterial nach Anspruch 5, worin der Überzug etwa 1/10 bis 5 Gew.-% des überzogenen Naht- oder Ligaturmaterials umfaßt.

8. Ein Verfahren zum Herstellen eines überzogenen Naht- oder Ligaturmaterials nach einem der Ansprüche 1 bis 7, umfassend
Auflösen des statistischen Copolymers in Aceton,
in Berührung bringen des Naht- oder Ligaturmaterials mit dem gelösten Copolymer,
Aufrechterhalten des Kontaktes zwischen dem Naht- oder Ligaturmaterial und dem gelösten Copolymer, bis das Copolymer auf dem Naht- oder Ligaturmaterial 1/10 bis 5 Gew.-% des überzogenen Naht- oder Ligaturmaterials umfaßt,
Entfernen des überzogenen Naht- oder Ligaturmaterials aus dem gelösten Copolymer und
Trocknen des Copolymers auf dem genannten Naht- oder Ligaturmaterial.

## Revendications

1. Suture ou ligature chirurgicale présentant de bonnes caractéristiques de sûreté du noeud et de remise en place du noeud, possédant un enrobage bioabsorbable comprenant un copolymère statistique comportant 70 à 85% en poids de motifs de formule (I) : 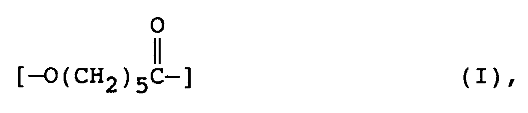 les motifs restants répondant à la formule (II) : 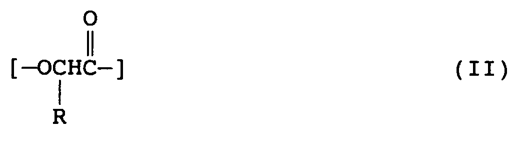 dans laquelle R est H ou CH₃.

2. Suture ou ligature selon la revendication 1, dans laquelle les motifs restants comprennent la formule (II) dans laquelle R est H.

3. Suture ou ligature selon la revendication 1, dans laquelle la viscosité inhérente dudit copolymère est de 0,2 à 1,4 dl/g (0,5 g/dl dans CHCl₃, 30°C).

4. Suture ou ligature selon la revendication 1, dans laquelle la suture ou la ligature est fabriquée à partir d'un polymère bioabsorbable préparé à partir d'un ou plusieurs monomères choisis dans le groupe constitué par les lactides, les carbonates et les lactones.

5. Suture ou ligature selon la revendication 3, dans laquelle la suture ou la ligature est fabriquée à partir d'un homopolymère préparé à partir du monomère glycolide, ou d'un copolymère préparé à partir du monomère glycolide et soit du monomère 1,3-dioxane-2-one, soit du monomère lactide, soit des deux.

6. Suture ou ligature selon la revendication 4, dans laquelle la suture ou la ligature est sous la forme de plusieurs filaments.

7. Suture ou ligature selon la revendication 5, dans laquelle l'enrobage constitue environ 0,10 à 5% en poids de la suture ou ligature enrobée.

8. Procédé de fabrication d'une suture ou ligature selon l'une quelconque des revendications 1 à 7, comprenant :
la dissolution dans l'acétone du copolymère statistique;
la mise en contact de la suture ou ligature avec le copolymère dissous;
le maintien du contact entre ladite suture ou ligature et le copolymère dissous jusqu'à ce que le copolymère sur ladite suture ou ligature constitue de 0,10 à 5% en poids de la suture ou ligature enrobée;
le retrait de ladite suture ou ligature enrobée dudit copolymère dissous; et
le séchage du copolymère sur ladite suture ou ligature.
